# EUROPEAN PATENT SPECIFICATION

(11) **EP 1 753 737 B1**
(45) Date of publication and mention of the grant of the patent: **16.07.2008**
(21) Application number: 05753173.3
(22) Date of filing: 17.05.2005
(51) Int. Cl.: C07D 301/26

(54) **PROCESS FOR PREPARING EPICHLOROHYDRIN FROM ETHANE**
VERFAHREN ZUR HERSTELLUNG VON EPICHLOROHYDRIN AUS ETHAN
PROCEDE DE PREPARATION D'EPICHLOROHYDRINE A PARTIR D'ETHANE

(30) Priority: 21.05.2004 US 573498 P
(43) Date of publication of application: 21.02.2007
(73) Proprietor: Dow Global Technologies Inc., Midland, MI 48674 (US)
(72) Inventor: MAUGHON, Bob, R., Midland, MI 48640 (US); FRYCEK, George, J., Midland, MI 48642 (US); JONES, Mark, E., Midland, MI 48642 (US)
(74) Representative: Raynor, John
(86) International application number: PCT/US2005/017192
(87) International publication number: WO 2005/116004

(56) References cited:
- EP-A- 1 059 278
- WO-A-01/38271
- GB-A- 2 173 496
- US-A- 3 557 229
- US-B1- 6 307 108
- DATABASE CROSSFIRE BEILSTEIN BEILSTEIN INSTITUT ZUR FOEDERUNG DER CHEMISCHEN WISSENSCHAFTEN, FRANKFURT AM MAIN, DE; Database-Accession no. 6948580 (REACTION ID) XP002351257 & JOURNAL OF THE AMERICAN CHEMICAL SOCIETY., vol. 70, 1948, page 2516, USAMERICAN CHEMICAL SOCIETY, WASHINGTON, DC.

## Description

The present invention relates to a process for preparing epichlorohydrin.

In the commercial processes for preparing ECH, ECH is made from allyl chloride, and allyl chloride is made from thermal chlorination of propylene using chlorine gas, a process that produces chlorinated by-products. Generally, chlorinated by-products are treated as waste material. A large amount of process water is used in the reaction of allyl chloride with additional chlorine to make propylene dichlorohydrin, the ECH intermediate. This process water must eventually be treated as waste.

EP-A-1059278 and GB-A-2173496 refer to the use of dichloropropanol mixtures for preparing ECH.

It would be desirable to provide a process to make epichlorohydrin which produces fewer halogenated by-products, and/or lower levels of halogenated contaminants by-products in the resulting resin or compound.

In a first aspect, the present invention is a process for preparing epichlorohydrin which comprises:
(1) converting ethane to 1,2-dichloroethylene (*cis*/*trans* mixture) in the presence of a catalyst,
(2) hydroformylating the 1,2-dichloroethylene in the presence of a catalyst, carbon monoxide, and hydrogen to produce 2,3-dichloropropanal;
(3) selectively hydrogenating the 2,3-dichloropropanal to 2,3-dichloropropanol in the presence of a catalyst ; and
(4) epoxidizing the 2,3-dichloropropanol with a base to produce epichlorohydrin.

The catalyst used in steps (1), (2) and (3) may be the same or different.

In a second aspect, the present invention is a process for preparing epichlorohydrin which comprises
(1) converting ethane to 1,2-dichloroethylene (*cis*/*trans* mixture) in the presence of a catalyst;
(2) adding MeOH to the 1,2-dichloroethylene to produce 2,3-dichloropropanol; and
(3) epoxidizing the 2,3-dichloropropanol with a base to produce epichlorohydrin.

In a third aspect, the present invention is a process for preparing epichlorohydrin which comprises
(1) converting ethane to 1,2-dichloroethylene (*cis*/*trans* mixture) in the presence of LaOCl catalyst;
(2) subjecting the 1,2-dichloroethylene to direct reductive hydroformylation in the presence of a reductive hydroformylation catalyst to produce 2,3-dichloropropanol; and
(3) epoxidizing the 2,3-dichloropropanol with a base to produce epichlorohydrin.

The process of the present invention can be represented by the following general equation:

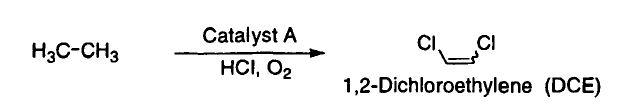

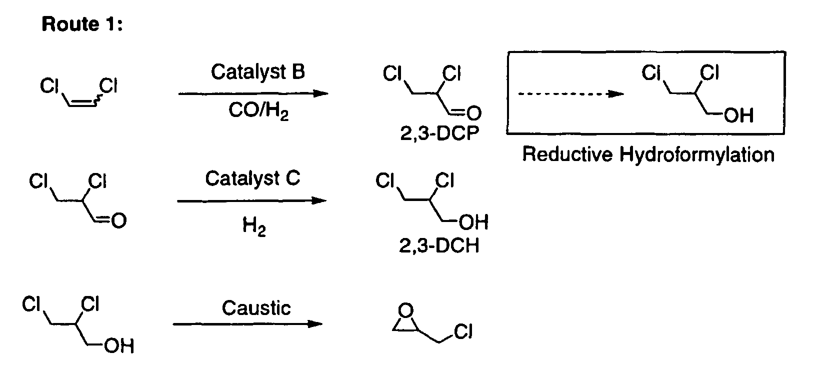

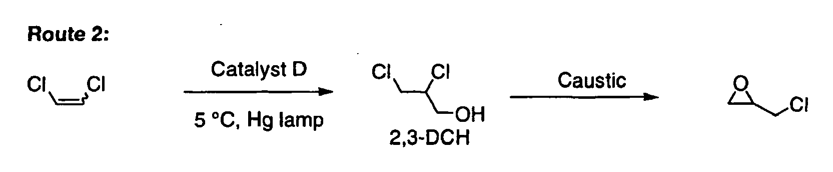

Dichloroethylenes can be made from ethane with the recycle of ethylene from the product stream. The process for producing vinyl chloride according to this first aspect includes the essential steps of: (a) combining reactants including ethylene, an oxygen source, and a chlorine source in a reactor containing a catalyst under conditions sufficient to produce a product stream comprising vinyl chloride, ethylene and hydrogen chloride; and (b) recycling ethylene in the product stream back for use in Step (a). The ethylene in question for Step (a) can be accompanied by ethane as a further hydrocarbon starting material, and can be comprised solely of recycled ethylene from the product stream, so that ethane in effect is alone used over time as the requisite, hydrocarbon feed. The catalyst utilized for this process in preferred embodiments may be characterized as a porous rare earth element- containing material (a "rare earth material"). Dichloroethylenes can be removed as a side stream in the production of vinyl chloride or vinyl chloride can be recycled to maximize the production of dichloroethylenes.

Suitable catalysts include rare earth oxychlorides of the formula MOCl, wherein M is at least one rare earth element chosen from lanthanum, cerium, neodymium, praseodymium, dysprosium, samarium, yttrium, gadolinium, erbium, ytterbium, holmium, terbium, europium, thulium, lutetium, or mixtures thereof. Most preferably, the salt is a porous, bulk lanthanum oxychloride (LaOCl) material. Under reaction conditions, the rare earth oxychloride. It is believed that the in situ formed catalyst comprises a chloride of the rare earth component present in the starting rare earth oxychloride. An example of such a chloride is MCl₃, wherein M is a rare earth component selected from lanthanum, cerium, neodymium, praseodymium, dysprosium, samarium, yttrium, gadolinium, erbium, ytterbium, holmium, terbium, europium, thulium, lutetium and mixtures thereof. Such catalysts are described in WO 0138273. Other suitable catalysts include supported copper catalysts, such as those described in GB1492945, GB 2009164 and US 5763710.

Preferably, the reactor is maintained between a temperature of greater than 350°C, more preferably greater than 375 degrees Celsius and a temperature less than 500°C, more preferably less than 450°C. Typically, the reactor is maintained between ambient pressure and 3.5 megapascals (MPa), gauge (500 pounds per square inch, gauge (psig)). Operation at pressure allows considerable flexibility to the down-stream processing operations, since higher pressure provides a driving force for the movement of materials into and through separation unit operations. Preferably, the operating pressure is between ambient and 2.1 MPa, gauge (300 psig), and most preferably between ambient and 1.1 MPa, gauge (150 psig). The process can be carried out in either a fixed bed or fluidized bed mode, though a fluidized process is preferred.

### Hydroformylation

Hydroformylation processes generally comprise contacting an olefinic moiety with a mixture of carbon monoxide and hydrogen in the presence of a hydroformylation catalyst and, optionally, in the presence of free organophosphorus ligand, under hydroformylation conditions sufficient to prepare an aldehyde. In the present invention, the hydroformylation process comprises contacting a reduced chain unsaturated acid or ester with carbon monoxide and hydrogen in the presence of a transition metal organophosphorus ligand complex catalyst and, optionally, free organophosphorus ligand, under hydroformylation conditions sufficient to prepare 2,3-dichloropropanal. The art fully describes catalysts and conditions for hydroformylation. See, for example, US-B1-6,307,108.

The catalysts useful in the hydroformylation process of this invention include any transition metal organophosphorus ligand complex catalyst that exhibits activity in hydroformylation processes. The suitable metals that make up the metal-organophosphorus ligand complexes includes the Group 8, 9, and 10 metals selected from rhodium (Rh), cobalt (Co), iridium (Ir), ruthenium (Ru), iron (Fe), nickel (Ni), palladium (Pd), platinum (Pt), osmium (Os), and mixtures thereof; with rhodium, cobalt, iridium, and ruthenium being preferred; rhodium, cobalt, and ruthenium being more preferred; and rhodium being most preferred. Other suitable metals include Group 11 metals selected from copper (Cu), silver (Ag), gold (Au), and mixtures thereof, as well as Group 6 metals selected from chromium (Cr), molybdenum (Mo), tungsten (W), and mixtures thereof. Mixtures of metals from Groups 6, 8, 9, 10, and 11 are also suitable.

The suitable organophosphorus ligands, which make up the free ligand and the ligand complexed in the transition metal-ligand complex catalyst, include, without limitation, organophosphines, for example, triorganophosphines; and organophosphites, for example, mono-, di-, and tri- organophosphites and bisphosphites. Other suitable organophosphorus ligands include, for example, organophosphonites, organophosphinites, organophosphorus amides, as well as mixtures of any of the aforementioned ligands. A wide variety of phosphorus ligand species are known in the art, as illustrated, for example, in US-B1-6,307,108.

The reaction conditions for the hydroformylation process encompassed by this invention include any conventional hydroformylation process conditions. For instance, the total gas pressure of hydrogen, carbon monoxide, and olefin starting compound may range from 1 psia (6.9 kPa) or greater to less than 10,000 psia (68,950 kPa). Preferably, the total gas pressure is less than 2,000 psia (13,800 kPa), and more preferably, less than 1,000 psia (6,850 kPa). More specifically, the carbon monoxide partial pressure of the hydroformylation process of this invention is preferably from greater than 1 psia (6.9 kPa), and preferably, greater than 3 psia (20.7 kPa). The carbon monoxide partial pressure is typically less than 1,000 psia (6,895 kPa), and preferably, less than 800 psia 5,516 (kPa). The hydrogen partial pressure is typically greater than 5 psia (34.5 kPa), preferably, greater than 10 psia (68.9 kPa). The hydrogen partial pressure is typically less than 500 psia (3,448 kPa), and preferably, less than 300 psia (2,069 kPa). In general, the H₂:CO molar ratio of gaseous hydrogen to gaseous carbon monoxide ranges from 1:10 to 10:1. The hydroformylation process is conducted typically at a reaction temperature greater than -25ºC, and preferably, greater than 50ºC. The hydroformylation process is conducted typically at a reaction temperature less than 150ºC, and preferably, less than 120ºC. The exact reaction time will depend upon the particular reactants and catalyst selected; but generally the reaction time is normally within a range of from 30 minutes to 200 hours. The hydroformylation process may be conducted in the presence of a solvent, suitable species of which include, without limitation, alkanes, cycloalkanes, aldehydes, ketones, ethers, esters, aromatics, alcohols.

### Hydrogenation

The hydrogenation of the present invention takes place in the presence of a heterogeneous transition metal-containing catalyst. The transition metal useful in the heterogeneous catalyst of the present invention may be one or more metals selected from any of Groups IB, IIB or IIIA-VIIIA on the periodic table of elements, as currently adopted by the International Union of Pure and Applied Chemistry (IUPAC). The catalyst metal is preferably selected from Group VIIIA of the IUPAC periodic table, including for example, iron, cobalt, nickel, ruthenium, rhodium, palladium, osmium, iridium, platinum and mixtures thereof. The catalyst metal is more preferably selected from the group consisting of ruthenium, iridium, rhodium, palladium, platinum, or mixtures thereof. The catalyst metal is most preferably selected from the group consisting of ruthenium, iridium or mixtures thereof.

An illustration of the catalyst of the present invention may be, for example, the iridium/ruthenium mixed metal catalysts disclosed in US Patent 6,350,922. The atomic ratio of iridium metal to ruthenium metal in the catalyst is generally from 0.02 to 15, preferably from 0.05 to 10, more preferably from 0.15 to 8, and most preferably from 0.3 to 2.0.

The heterogeneous catalysts useful in the present invention may be, for example, a transition metal deposited or absorbed on an insoluble support such as silica, silylated silica, carbon, alumina, titania, zirconia, magnesia and other common supports known in the art as described in Poncelet et al. editors, Preparation of Catalysts III, New York, 1983; P.N. Rylander, Hydrogenation Methods, Academic Press, London, 1985; P.N. Rylander, Catalytic Hydrogenation Over Platinum Metals, Academic Press, New York, 1967; P. Rylander, Catalytic Hydrogenation in Organic Syntheses, Academic Press, New York, 1979. The heterogeneous catalyst of the present invention may also be a transition metal coordinated to ligands bonded to a resin, for example ruthenium on phosphinated polystyrene. The catalyst is typically in the form of granules or pellets. The amount of active catalyst on a support is generally from 0.1 percent (percent) to 25 percent and preferably from 0.5 percent to 15 percent.

One advantage of using a heterogeneous catalyst in the process of the present invention is the ability to separate the catalyst from the reaction solution by various means such as by filtration.

The ideal ratio of catalyst to reagents used in the present process varies depending upon flow rate, bed size, temperature, desired conversion, reagents and other factors of the present process. Usually, a heterogeneous catalyst bed contains 0.0001 mole to 100 moles of catalyst metal for each mole of α-haloketone which passes through the bed per hour.

The dihalopropanal is hydrogenated by reacting the dihalopropanal with a hydrogenating agent in the presence of a catalyst. The hydrogenation reaction is described in detail in U.S. Pat. No. 5,744,655. The hydrogenating agent useful in the present invention may be, for example, molecular hydrogen, alcohols, hydrazines, formates or combinations thereof. Examples of suitable alcohols useful as the hydrogenating agent in the present invention can be primary or secondary alcohols such as methanol, ethanol and C₃ to C₁₀ primary and secondary alcohols. Examples of other secondary alcohols useful in the present invention are described in U.S. Pat. No. 2,860,146. The hydrogenating agent used in the present invention is preferably molecular hydrogen.

The hydrogenation reaction consumes one mole of hydrogenating agent per mole of dihalopropanol which is made. Generally, at least 0.6 moles of hydrogenating agent per mole of 2,3-dihalopropanal are available to be consumed during the course of the reaction, preferably at least 0.75 moles of molecular hydrogen per mole of 2,3-dihalopropanal are available to be consumed during the course of the reaction, more preferably at least 0.9 moles and most preferably at least 1 mole are available to be consumed during the course of the reaction. When less than 1 mole of hydrogenating agent per mole of 2,3-dihalopropanal is available to be consumed during the course of the reaction, the reaction is less efficient because complete conversion of the 2,3-dihalopropanal is not obtained. However, not all of the hydrogenating agent need be available at the start of the reaction. The hydrogenating agent may be added step-wise or continuously as the reaction progresses. In this case, the reaction mixture at any one time may contain a stoichiometric excess of dihalopropanal over hydrogenating agent. As one embodiment of the present invention, an excess of hydrogenating agent required may be used for completing the conversion in the reaction. Generally, for example, from 10 percent to 20 percent excess hydrogenating agent may be used.

The maximum quantity of hydrogenating agent source is not critical and is governed by practical considerations such as pressure, reactor efficiency and safety. When the hydrogenating agent source is gaseous, then the quantity of hydrogenating agent is preferably at least enough to provide the desired pressure. However, in most cases, the reactor preferably contains no more than 1,000 moles of molecular hydrogen per mole of 2,3-dihalopropanal and more preferably contains no more than 100 moles. Gaseous hydrogenating agent sources, such as molecular hydrogen, are preferably used according to known methods for mixing a gaseous reagent with a liquid reaction mixture, such as bubbling the gas through the mixture with agitation or solubilizing the hydrogen under pressure.

The reaction of the present invention is optionally, but preferably, carried out in the presence of a solvent. The solvent used is preferably inert with respect to all of the reagents under the reaction conditions. The solvent may be selected such that: (1) the solvent does not boil under reaction conditions; and (2) the α-haloalcohol can be recovered from the solvent, for example by distillation, extraction, or any other known recovery means.

Examples of suitable solvents useful in the present invention include aromatic and aliphatic hydrocarbons, chlorinated hydrocarbons, ethers, glymes, glycol ethers, esters, alcohols, amides, and mixtures thereof. Specific examples of the solvents useful in the present invention include toluene, cyclohexane, hexane, methylene chloride, dioxane, dimethyl ether, diglyme, 1,2-dimethoxyethane, ethyl acetate, methanol, NMP, and mixtures thereof. The quantity of solvent used in the present invention is not critical and is governed primarily by practical considerations, such as the efficiency of the reactor. Generally, the amount of the solvent present in the reaction mixture ranges from 0 to 99.99 weight percent.

Unless otherwise indicated, all parts and percentages are by weight.

### EXAMPLE 1: CO-PRODUCTION OF VINYL CHLORIDE AND 1,2-DICHLOROETHYLENE FROM A STREAM COMPRISING ETHANE

A porous, refractory composition comprising lanthanum was prepared by precipitating a solution of LaCl₃ in water with ammonium hydroxide to neutral pH causing the formation of a gel. The mixture was filtered, and the solid was washed. The solid was resuspended and spray-dried to yield particles with a 60 micron nominal diameter. The collected powder was subsequently calcined at 550°C in air. The resulting solid was compressed, crushed and sieved to yield particles suitable for additional laboratory reactor testing. This procedure produced a solid matching the X-ray powder diffraction pattern of LaOCl. The particles were placed in a pure nickel (alloy 200) reactor. The reactor was configured such that ethylene, ethane, HCl, O₂ and inert gas (He and Ar mixture) could be fed to the reactor. The function of the argon was as an internal standard for the analysis of the reactor feed and effluent by gas chromatography. The results are shown in Table I.

**TABLE I: Results of Ethane Oxychlorination Experiment**

| Molar feed ratios | | | | | | | Conversions | | | | Selectivities | | | | | | |
|---|---|---|---|---|---|---|---|---|---|---|---|---|---|---|---|---|---|
| C₂H₄ | C₂H₆ | HCl | He+Ar | O₂ | sccm | T(°C) | C₂H₄ | C₂H₆ | HCl | O₂ | C₂H₄ | CO | CO₂ | EDC | EtCl | VCM | DCE |
| 0.00 | 0.07 | 1.00 | 0.16 | 1 | 19.7 | 399 | 0% | 96% | 16% | 15% | 3% | 19% | 2% | 26% | 0% | 10% | 32% |

### EXAMPLES 2-6

### General Procedure:

Heterogeneous catalysts were prepared by impregnation of silica (Davison 57) with aqueous or alcohol-based metal salt solutions of IrCl₃•3H₂O and one of the following: RuCl₃•H₂O, (NH₄)₆Mo₇O₂₄•4H₂O, MnCl₂•4H₂O, OsCl₃•3H₂O, or FeCl₃•6H₂O. The mixed metal systems could be prepared by coimpregnation or by impregnation of one metal salt (and dried) followed by the other. The catalysts were air dried and then prereduced under dynamic H₂/N₂ (5 percent hydrogen) at 200°C. The catalysts were then stored and handled in an inert atmosphere glove box.

A 300-mL Parr reactor vessel was loaded with a catalyst charge and the reactor vessel was evacuated and nitrogen flushed three times. A solvent/2,3-dichloropropanal (DCP) mixture was sparge degassed with nitrogen and added to the Parr reactor with a syringe. The reactor was pressurized/vented to 250/20 psig (1.7 mPa/138 kPa) nitrogen and 1000/20 psig (6.9 mPa/138 kPa) hydrogen, then placed under 1000 psig (6.9 mPa) hydrogen and heated to 65°C to 100°C. Samples were removed by syringe after venting the reactor to less than 15 psig (103 kPa).

Samples were analyzed by gas chromatography (GC) using a Hewlett Packard HP-5890 gas chromatograph equipped with a 25 m HP-5 Ultra 2 capillary column with split injection. Approximately 120 µL of the reaction mixture was dissolved in 5.0 mL of chloroform which contained a known amount of chlorobenzene as a GC standard (typically 0.05 weight percent). "Selectivity" is defined as the molar ratio of the amount of 2,3-dihalopropanol formed to the amount of 2,3-dihalopropanal consumed.

A comparison of activity and selectivity over two successive batch cycles for pure ruthenium/silica, pure iridium/silica, and selected ruthenium/iridium/silica catalysts is presented in Table I below. The ability of the ruthenium/iridium catalysts to provide sustained hydrogenation activity with high selectivity superior to either the pure ruthenium or pure iridium catalysts is demonstrated.

The pure iridium catalyst (Example 6) has good initial activity (79.3 percent conversion in 30 minutes) with high selectivity (>99 percent), but the conversion for the second charge (Run #2) is poor (7.9 percent conversion in 30 minutes; 90 percent loss of activity).

The pure ruthenium catalyst (Example 5) shows stable performance over two cycles (Run #1 and Run #2) with 20 percent loss in activity, but the activity of the ruthenium catalyst is low (24.7 percent and 19.7 percent conversion in 30 minutes) respectively.

The combination of iridium and ruthenium metal in catalysts of the present invention (Examples 2, 3 and 4) provides in all cases very good selectivity (>90 percent) to 2,3-dihalopropanols. In addition, catalysts of the present invention show activities which in all cases are better than that observed for pure ruthenium (at least >47 percent vs. 24.7 percent and 19.7 percent); and the loss of activity between cycles (Runs #1 and Runs #2) for the catalysts of the present invention are superior to the pure iridium catalyst (32 percent, 33 percent, 13 percent all less than 90 percent).

In addition, the loss of activity for the catalysts of the present invention are comparable to, and in Example 4, superior to that of the pure ruthenium catalyst (13 percent; less than 20 percent).

**Table II**

| Example | Run # | Catalyst Composit ion | g DCP^{a}/g Catalyst | Temperat ure (°C) | Time (minutes ) | Conversi on (%) | Δ% Loss of Activ ity | Selectivity (%) |
|---|---|---|---|---|---|---|---|---|
| Example 2 | 1 | 2.0% Ru/0.5% Ir/Silica | 2.52 | 85 | 30 | 68.9 | 32 | 92.3 |
| | 2 | " | 2.51 | 84 | 30 | 47.2 | | 90.8 |
| Example 3 | 1 | 1.5% Ru/1.0% Ir/Silica | 2.50 | 86 | 30 | 85.6 | 33 | 97.8 |
| | 2 | " | 2.49 | 84 | 30 | 57.2 | | 94.2 |
| Example 4 | 1 | 0.5% Ru/2.0% Ir/silica | 2.50 | 84 | 30 | 100 | 13 | 98.6 |
| | 2 | " | 2.50 | 85 | 30 | 87.4 | | 98.2 |
| Example 5 | 1 | 2.5% Ru/Silica | 2.49 | 85 | 30 | 24.7 | 20 | 90.9 |
| | 2 | " | 2.50 | 85 | 30 | 19.7 | | 90.6 |
| Example 6 | 1 | 2.5% Ir/Silica | 2.48 | 85 | 30 | 79.3 | 90 | >99 |
| | 2 | " | 2.50 | 86 | 30 | 7.9 | | >99 |

| | | | | | | | | |
|---|---|---|---|---|---|---|---|---|
| ^{a} DCP = dichloropropanal ^{b} Parr reactor in cyclohexane under ∼ 1000 psig H₂. | | | | | | | | |

## Claims

1. A process for preparing epichlorohydrin which comprises:
(1) converting ethane to 1,2-dichloroethylene (*cis*/*trans* mixture) in the presence of a catalyst;
(2) hydroformylating the 1,2-dichloroethylene in the presence of a catalyst, carbon monoxide, and hydrogen to produce 2,3-dichloropropanal;
(3) selectively hydrogenating the 2,3-dichloropropanal to 2,3-dichloropropanol in the presence of a catalyst; and
(4) epoxidizing the 2,3-dichloropropanol with a base to produce epichlorohydrin.

2. The process of Claim 1 wherein the hydroformylation catalyst is a transition metal-organophosphorus ligand complex catalyst.

3. The process of Claim 1 wherein the hydrogenation catalyst is heterogeneous transition metal-containing catalyst.

4. The process of Claim 3 wherein the hydrogenation catalyst is iridium/ruthenium mixed metal catalyst.

5. The process of Claim 1 wherein the hydroformylation reaction is conducted in the presence of a solvent.

6. The process of Claim 1 wherein the hydroformylation reaction is conducted for about 30 minutes at a temperature of less than 150°C.

7. A process for preparing epichlorohydrin which comprises
(1) converting ethane to 1,2-dichloroethylene (*cis*/*trans* mixture) in the presence of a catalyst;
(2) adding MeOH to the 1,2-dichloroethylene to produce 2,3-dichloropropanol; and
(3) epoxidizing the 2,3-dichloropropanol with caustic to produce epichlorohydrin.

8. A process for preparing epichlorohydrin which comprises
(1) converting ethane to 1,2-dichloroethylene (*cis*/*trans* mixture) in the presence of a catalyst;
(2) subjecting the 1,2-dichloroethylene to direct reductive hydroformylation in the presence of a reductive hydroformylation catalyst to produce 2,3-dichloropropanol; and
(3) epoxidizing the 2,3-dichloropropanol with caustic to produce epichlorohydrin.

## Patentansprüche

1. Verfahren zur Herstellung von Epichlorhydrin, mit den folgenden Schritten:
(1) Umwandeln von Ethan in 1,2-Dichlorethylen (*cis*/*trans*-Mischung) in Gegenwart eines Katalysators;
(2) Hydroformylieren von 1,2-Dichlorethylen in Gegenwart eines Katalysators, Kohlenmonoxid, und Wasserstoff, um 2,3-Dichlorpropanal zu erzeugen;
(3) wahlweises Hydrieren von 2,3-Dichlorpropanal zu 2,3-Dichlorpropanol in Gegenwart eines Katalysators; und
(4) Epoxidieren von 2,3-Dichlorpropanol mit einer Base, um Epichlorhydrin zu erzeugen.

2. Verfahren nach Anspruch 1, wobei der Hydroformylierungskatalysator ein Katalysator aus einem Übergangsmetall-Organophosphorligand-Komplex ist.

3. Verfahren nach Anspruch 1, wobei der Hydrierungskatalysator ein heterogener übergangsmetallhaltiger Katalysator ist.

4. Verfahren nach Anspruch 3, wobei der Hydrierungskatalysator ein Iridium/Ruthenium-Mischmetalikatalysator ist.

5. Verfahren nach Anspruch 1, wobei die Hydroformylierungsreaktion in Gegenwart eines Lösungsmittels durchgeführt wird.

6. Verfahren nach Anspruch 1, wobei die Hydroformylierungsreaktion für etwa 30 Minuten bei einer Temperatur von weniger als 150°C durchgeführt wird.

7. Verfahren zur Herstellung von Epichlorhydrin, mit den folgenden Schritten:
(1) Umwandeln von Ethan in 1,2-Dichlorethylen (*cis*/*trans*-Mischung) in Gegenwart eines Katalysators;
(2) Zugabe von MeOH zu dem 1,2-Dichlorethylen, um 2,3-Dichlorpropanol zu erzeugen; und
(3) Epoxidieren von 2,3-Dichlorpropanol mit Ätznatron, um Epichlorhydrin zu erzeugen.

8. Verfahren zur Herstellung von Epichlorhydrin, mit den folgenden Schritten:
(1) Umwandeln von Ethan in 1,2-Dichlorethylen (*cis*/*trans*-Mischung) in Gegenwart eines Katalysators;
(2) das 1,2-Dichlorethylen wird in Gegenwart eines reduktiven Hydroformylierungskatalysators einer direkten reduktiven Hydroformylierung unterzogen, um 2,3-Dichlorpropanol zu erzeugen; und
(3) Epoxidieren von 2,3-Dichlorpropanol mit Ätznatron, um Epichlorhydrin zu erzeugen.

## Revendications

1. Procédé de préparation d'épichlorhydrine, comportant les étapes suivantes :
1) convertir de l'éthane en 1,2-dichloro-éthylène à l'état de mélange d'isomères cis et trans, en présence d'un catalyseur ;
2) soumettre ce 1,2-dichloro-éthylène à une hydroformylation, en présence d'un catalyseur, de monoxyde de carbone et d'hydrogène, de manière à produire du 2,3-dichloro-propanal ;
3) hydrogéner sélectivement ce 2,3-dichloro-propanal, en présence d'un catalyseur, pour en faire du 2,3-dichloro-propanol ;
4) et convertir ce 2,3-dichloro-propanol en époxyde au moyen d'une base, pour produire de l'épichlorhydrine.

2. Procédé conforme à la revendication 1, dans lequel le catalyseur d'hydroformylation est un catalyseur de type complexe de métal de transition et de ligand organophosphoré.

3. Procédé conforme à la revendication 1, dans lequel le catalyseur d'hydrogénation est un catalyseur pour catalyse hétérogène, contenant un métal de transition.

4. Procédé conforme à la revendication 3, dans lequel le catalyseur d'hydrogénation est un catalyseur à métaux associés iridium/ruthénium.

5. Procédé conforme à la revendication 1, dans lequel la réaction d'hydroformylation est effectuée en présence d'un solvant.

6. Procédé conforme à la revendication 1, dans lequel la réaction d'hydroformylation est effectuée en un laps de temps d'environ 30 minutes et à une température inférieure à 150 °C.

7. Procédé de préparation d'épichlorhydrine, comportant les étapes suivantes :
1) convertir de l'éthane en 1,2-dichloro-éthylène à l'état de mélange d'isomères cis et trans, en présence d'un catalyseur ;
2) additionner du méthanol sur ce 1,2-dichloro-éthylène, de manière à produire du 2,3-dichloro-propanol ;
3) et convertir ce 2,3-dichloro-propanol en époxyde au moyen d'une base caustique, pour produire de l'épichlorhydrine.

8. Procédé de préparation d'épichlorhydrine, comportant les étapes suivantes :
1) convertir de l'éthane en 1,2-dichloro-éthylène à l'état de mélange d'isomères cis et trans, en présence d'un catalyseur ;
2) soumettre ce 1,2-dichloro-éthylène à une hydroformylation réductrice, en présence d'un catalyseur d'hydroformylation réductrice, de manière à produire directement du 2,3-dichloro-propanol ;
3) et convertir ce 2,3-dichloro-propanol en époxyde au moyen d'une base caustique, pour produire de l'épichlorhydrine.
